(19) Europäisches Patentamt
European Patent Office
Office européen des brevets

(11) Veröffentlichungsnummer: **0 075 925**
**B1**

(12) **EUROPÄISCHE PATENTSCHRIFT**

(45) Veröffentlichungstag der Patentschrift: **02.01.91**

(51) Int. Cl.$^5$: **A 61 K 35/16**

(21) Anmeldenummer: **82108965.3**

(22) Anmeldetag: **28.09.82**

(54) **Verfahren zur Herstellung eines spezifisch auf das Ernährungszentrum wirkenden appetitregelnden Wirkstoffes sowie der nach diesem Verfahren erhältliche Wirkstoff.**

(30) Priorität: **28.09.81 HU 278381**
**03.03.82 HU 278381**

(43) Veröffentlichungstag der Anmeldung:
**06.04.83 Patentblatt 83/14**

(45) Bekanntmachung des Hinweises auf die Patenterteilung:
**02.01.91 Patentblatt 91/01**

(84) Benannte Vertragsstaaten:
**AT BE CH DE FR GB IT LI NL SE**

(56) Entgegenhaltungen:
**FR-A-2 434 818**

**CHEMICAL ABSTRACTS, Band 98, Nr. 15, 11. April 1983, Seite 88, abstract 119817k, Columbus, Ohio, US; N. JANOS et al.:"An improved method for the preparation of highly purified satietin samples from human serum"**

(73) Patentinhaber: **RICHTER GEDEON VEGYESZETI GYAR R.T.**
**Gyömröi ut 19-21**
**H-1475 Budapest X (HU)**

(72) Erfinder: **Knoll, József, Dr.**
**Jászai M. tér 4/b**
**Budapest XIII (HU)**
Erfinder: **Nagy, János, Dr., Dipl. Chem.**
**Jókai u. 2**
**Szentendre (HU)**
Erfinder: **Kalász, Huba, Dr., Dipl. Chem.**
**Hevesi Gy. u. 95**
**Budapest XIV (HU)**
Erfinder: **Knoll, Berta, Dr.**
**Madách t. 2-6**
**Budapest VII (HU)**

(74) Vertreter: **Beszédes, Stephan G., Dr.**
**Patentanwalt**
**Münchener Strasse 80a Postfach 1168**
**D-8060 Dachau (DE)**

**EP 0 075 925 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

(56) Entgegenhaltungen:
CHEMICAL ABSTRACTS, Band 98, Nr. 17, 25. April 1983, Seite 119, abstract 13847h, Columbus, Ohio, US; N. JANOS et al.:"Satietin: an endogenous selective appetite regulator. Separation an chemical characterization"

CHEMICAL ABSTRACTS, Band 97, Nr. 15, 11. Oktober 1982, Seite 133, abstract 121091s, Columbus, Ohio, US; J. KNOLL:"Satietin: endogenous regulation of food intake", ADV. BIOCHEM. PSYCHOPHARMACOL. 1982, 33

CHEMICAL ABSTRACTS, Band 96, Nr. 21, 24. Mai 1982, Seite 85, abstract 174490g, Columbus, Ohio, US; J. KNOLL:"Anorectic agents and satietin, an endogenous inhibitor of food intake", ADV. PHARMACOL. THER. PROC. INT. CONGR. 1981, 1, 147-62

CHEMICAL ABSTRACTS, Band 91, Nr. 25, 17. Dezember 1979, Seite 393, abstract 208025y, Columbus, Ohio, US; J. KNOLL:"Satietin: a highly potent anorexogenic substance in human serum", PHYDIOL. BEHAV. 1979, 23(3), 497-502
NEUROSCIENCE LETTERS, Band Suppl. Nr. 1, 17. November 1978, Seite S55; J. KNOLL:"New aspects in the chemoregulation of food intake"

**Beschreibung**

Die Erfindung betrifft ein Verfahren zur Herstellung eines spezifisch auf das Ernährungszentrum wirkenden appetitregelnden Wirkstoffes aus menschlichem und/oder tierischem Blutserum sowie den erhaltenen Wirkstoff zur Verwendung als Appetitregler.

Aus der ungarischen Patentschrift 178 703 ist bereits ein spezifisch auf das Ernährungszentrum wirkender appetitregelnder Wirkstoff, der aus menschlichem und/oder tierischem Blut isoliert wird, bekannt. Gemäß dem in dieser Druckschrift beschriebenen Verfahren wird menschliches und/oder tierisches Blutserum durch ein bis zu einem Molekulargewicht von 50 000 durchlässiges Membranfilter filtriert, das Filtrat eingedampft, der Rückstand in Wasser gelöst und die Lösung an einem Gel (Ausschlußvolumen unter M = 50 000 Dalton) chromatographiert. Es wird mit einer 0,1 bis 1,0 Gew.-% Natriumchlorid enthaltenden neutral reagierenden wäßrigen Lösung eluiert. Das Eluat wird fraktioniert aufgefangen, die biologisch aktiven Fraktionen werden eingedampft, der Rückstand wird in der erforderlichen Menge Wasser gelöst, die Lösung wird erneut an einem Gel der genannten Art chromatographiert und mit Wasser fraktioniert eluiert und die eine biologische Aktivität aufweisenden Fraktionen werden eingedampft. Auf diese Weise konnte eine durch Hydrolyse in Aminosäure- und Zuckerbestandteile (etwa 60 Gew.-% Aminosäuren und 10 Gew.-% Zucker) trennbare als Glykoproteid scheinende Fraktion abgetrennt werden, die sich als ein spezifisch auf das Ernährungszentrum wirkender appetitregelnder Wirkstoff, der auf das Zentralnervensystem weder anregend noch depressiv wirkt, erwies.

Die appetitregelnde Wirksamkeit dieses Wirkstoffes genügt aber noch nicht allen Anforderungen.

Der Erfindung liegt daher die Aufgabe zugrunde, ein Verfahren zur Herstellung eines spezifisch auf das Ernährungszentrum wirkenden appetitregelnden Wirkstoffes aus menschlichem und/oder tierischem Blutserum, durch welches ein reinerer Wirkstoff mit besserer appetitregelnder Wirkung erhalten werden kann, sowie, den erhaltenen Wirkstoff zur Verwendung als Appetitregler zu schaffen.

Das Obige wurde überraschenderweise durch die Erfindung erreicht.

Es wurde nämlich nun überraschenderweise festgestellt, daß die gemäß dem genannten bekannten Verfahren herstellbare Fraktion mittels eines neuen Verfahrens weiter zerlegt und so ein neues Produkt, welches 3- bis 4-mal so wirksam wie die nach dem bekannten Verfahren abgetrennte Fraktion ist, erhalten werden kann. Die chemische Zusammensetzung dieses Produktes weicht von der der bekannten Fraktion wesentlich ab. Das Produkt ist als physikalisch homogene, chemisch reine Substanz definierter Zusammensetzung anzusehen.

Dabei wurde festgestellt, daß der Eiweiß- beziehungsweise Peptidgehalt der nach dem genannten bekannten Verfahren abgetrennten Wirkstofffraktion zu einem bedeutenden Teil in chemisch nicht gebundener oder nur locker gebundener Form als hinsichtlich der Satietinwirkung inerter Bestandteil vorliegt und mittels entsprechender Eiweißfällverfahrensweisen vom eigentlichen Wirkstoff abgetrennt werden kann. Die danach noch zurückbleibenden niedermolekularen, peptidartigen und sonstigen Begleitstoffe können durch weitere Reinigungsstufen, nämlich mittels Elektrophorese und mittels Affinitätschromatographie, völlig entfernt werden. Auf diese Weise ist der reine chemisch einheitliche Satietinwirkstoff herstellbar.

Gegenstand der Erfindung ist daher ein Verfahren zur Herstellung eines spezifisch auf das Ernährungszentrum wirkenden appetitregelnden Wirkstoffes aus menschlichem und/oder tierischem Blutserum durch dessen Membranfiltrieren durch ein bis zu einem Molekulargewicht von 50 000 durchlässiges Membranfilter und Eindampfen des Filtrates sowie Gelchromatographieren einer Lösung des erhaltenen Materiales an einem Gel mit einem Ausschlußvolumen M unter 50 000 Dalton und fraktioniertes Eluieren mit einer wäßrigen Lösung, erneutes Gelchromatographieren einer Lösung der konzentrierten biologisch aktiven Fraktionen an einem Gel mit einem Ausschlußvolumen M unter 50 000 Dalton und fraktioniertes Eluieren mit Wasser und Konzentrieren der biologisch aktiven Fraktionen, welches dadurch gekennzeichnet ist, daß das beim Membranfiltrieren erhaltene Filtrat nur partiell eingedampft (eingeengt) wird und aus dem erhaltenen Konzentrat der unlösliche Teil entfernt wird, dann zur flüssigen Phase bei 0 bis 10°C eine wäßrige Trichloressigsäurelösung bis zum Erreichen einer Trichloressigsäurekonzentration von 5 bis 25 Gew./Vol.-% zugegeben wird und das ausgefallene Eiweiß entfernt wird sowie das Gelchromatographieren der erhaltenen Lösung an einem Gel mit einem Ausschlußvolumen unter M = 4 000 Dalton durchgeführt wird und das Eluieren mit einer 0,5 bis 1,0 gew.-%-igen wäßrigen Natriumchloridlösung oder einer wäßrigen Pufferlösung, insbesondere Phosphatpufferlösung, mit einem pH-Wert von 6,0 bis 7,0 vorgenommen wird, das Konzentrieren der biologisch aktiven Fraktionen durch Lyophilisieren bewerkstelligt wird, das erneute Gelchromatographieren des erhaltenen Konzentrates an einem Gel mit einem Ausschlußvolumen unter M = 3 000 Dalton durchgeführt wird und das Konzentrieren der mit Wasser eluierten biologisch aktiven Fraktionen durch Lyophilisieren vorgenommen wird sowie das erhaltene Produkt einer weiteren Reinigung in der Weise unterzogen wird, daß eine bei einem pH-Wert von 6,0 bis 6,5 vorgenommene Elektrophorese durchgeführt wird und das an der Startstelle gebliebene Hauptprodukt abgetrennt und unter Verwendung eines in Bezug auf Glucopyranose- und/oder Mannopyranosegruppen eine spezifische Affinität zeigenden Adsorbens einer Affinitätschromatographie unterzogen wird.

Der Begriff "Ausschlußvolumen", auch "Ausschlußgrenze" genannt, ist wie folgt zu verstehen:

Das Gel besteht aus Kunststoffkörnern mit feinen Poren. Die Stoffe mit einem Molekulargewicht unter

dem Ausschlußvolumwert dringen in diese Poren ein und werden dort in einer ihr Auswaschen verzögernden Weise festgehalten, während die Stoffe mit einem Molekulargewicht über dem Ausschlußvolumwert an der Oberfläche der Körner verbleibend und von dieser schnell fortgewaschen werden.

Vorzugsweise wird das Entfernen des unlöslichen Teiles des nach dem partiellen Eindampfen des Filtrates des Membranfiltrierens erhaltenen Konzentrates durch Zentrifugieren durchgeführt.

Es ist auch bevorzugt, die Zugabe der Trichloressigsäurelösung bis zum Erreichen einer Konzentration von 10 bis 12 Gew./Vol.-% vorzunehmen.

Ferner ist es bevorzugt, das Entfernen des auf die Zugabe der Trichloressigsäurelösung hin ausgefallenen Eiweißes durch Ultrazentrifugieren durchzuführen.

Zum Eluieren kann eine Natriumchloridlösung, solche mit einer Konzentration von 0,1 bis 1,0 Gew.-%, vorzugsweise 0,9 Gew.-%, verwendet werden.

Das erfindungsgemäße Verfahren wird zweckmäßig wie folgt durchgeführt.

Die erste Reinigungsstufe des erfindungsgemäßen Verfahrens, des Membranfiltrieren (Ultrafiltrieren), kann zum Beispiel mit Amicon® UM-10- oder Sartorius®-Membranen, vorteilhaft unter einem Druck von 3 atm unter ständigem Rühren, vorgenommen werden. Das erhaltene Filtrat wird vorteilhaft durch Vakuumeindampfen partiell eingedampft und somit konzentriert und der unlösliche Teil wird vorzugsweise durch Zentrifugieren entfernt. Die erhaltene trübe, aber niederschlagfreie Lösung wird auf 0 bis 10°C gekühlt und dann mit einer wäßrigen Trichloressigsäurelösung bis zu einer Konzentration von 5 bis 25 Gew./Vol.-%, vorzugsweise 10 bis 12 Gew./Vol.-%, insbesondere etwa 10 Gew./Vol.-%, versetzt. Die Lösung, in der sich ein Niederschlag ausscheidet, wird bei 0 bis 5°C mindestens 1 Stunde, vorzugsweise jedoch über Nacht, stehengelassen und dann wird das gefällte Eiweiß bei der gleichen Temperatur durch Ultrazentrifugieren abgetrennt, wobei eine klare lebhaft gelbe Lösung erhalten wird. Durch die Behandlung mit der Trichloressigsäurelösung werden sämtliche hochmolekularen Serumeiweiße einschließlich des Albumines entfernt. Das einen hohen Kohlenhydratgehalt aufweisende Satietin fällt hingegen nicht aus, sondern bleibt in Lösung, obwohl es noch immer eine geringe Eiweißmenge enthält. Daraus folgt, daß diese Eiweißmenge bereits in chemisch gebundener Form vorliegen muß, und zwar an den Kohlenhydratteil des Satietines.

Die nächste Reinigungsstufe ist die präparative Gelchromatographie an einem Gel mit einem Ausschlußvolumen unter M = 4 000 Dalton. Vorzugsweise werden mit Sephadex® G-15 oder Sephadex® G-25 gefüllte Säulen verwendet. Zum Eluieren wird vorzugsweise eine wäßrige 0,1 m Ammoniumacetatpufferlösung mit einem pH-Wert von 6,6 verwendet. Wie es sich in Versuchen erwies, konnten mit dieser Pufferlösung die Fraktionen mit Satietinaktivität am wirksamsten abgetrennt werden und infolge der Flüchtigkeit des Puffers wird ein praktisch salzfreies Produkt erhalten. Außerdem ist die Verwendung einer 0,9%-igen physiologischen Natriumchloridlösung oder von verschiedenen Phosphatpufferlösungen mit pH-Werten von 6,0 bis 7,0 vorteilhaft. Die aktive Fraktion erscheint beim Ausschlußvolumen der Gelsäule ($V_o$, bei welchem $K_d$, der Verteilungskoeffizient der untersuchten Substanz, gleich 0 ist). Das bedeutet, daß die Moleküle des Produktes größer sind als die inneren Porendurchmesser des Geles und deshalb nicht in das Innere des Geles eindringen können. Sie sind vom Gel ausgeschlossen und erscheinen zusammen mit dem Eluiermittel in der Eluatfraktion, die etwa $\frac{1}{3}$ des Volumens der Säule entspricht. An Sephadex® G-15 verhalten sich beispielsweise die Moleküle mit einem Molekulargewicht über 1 500 in der geschilderten Weise. Die aus dem Serum stammenden Substanzen mit einem geringeren Molekulargewicht hingegen dringen in das Innere des Geles ein und werden dort verzögert (in diesem Falle ist $K_d$ größer als 0). Da die Menge der im Ultrafiltrat enthaltenen niedermolekularen Stoffe zusammen mit den Salzen unvergleichbar größer als die Satietinmenge ist, entspricht die Reinigungswirksamkeit dieser Reinigungsstufe ganzen Größenordnungen. Die zum Fällen der Eiweiße verwendete Trichloressigsäure wird als niedermolekulare Verbindung in der Gelsäule zurückgehalten. Im $\frac{1}{3}$ des Säulenvolumens entsprechenden Elutionsbereich (Ausschlußbereich) wird salz- und säurefreier Wirkstoff erhalten. Diese Fraktionen werden vereinigt und durch Lyopholisieren konzentriert und gegebenenfalls wird das Konzentrat zu dessen Lösen mit wenig Wasser versetzt.

Die folgende Reinigungsstufe ist ebenfalls gelchromatographisch, es wird ein Gel mit einem Ausschlußvolumen unter M = 3 000 Dalton verwendet. Vorzugsweise wird als Gel Bio-Gel P-2® [hergestellt von der Firma Bio-Rad Laboratories, Richmond, California, USA] verwendet und als Wasser für das Eluiermittel destilliertes Wasser eingesetzt. In dieser Reinigungsstufe werden die im Produkt noch enthaltenen Salze und niedermolekularen Fragmente, zum Beispiel Bruchstücke von Peptiden, entfernt. Auch in dieser Stufe werden die Fraktionen mit Satietinaktivität vom Gel nicht aufgenommen und erscheinen im $\frac{1}{3}$ des Säulenvolumens entsprechenden Eluatvolumen. Durch Vereinigen der biologisch aktiven Fraktionen und Lyophilisieren wird der Wirkstoff in Form eines gut zu handhabenden blaßgelben Pulvers erhalten. Aus 1 l menschlichem Blutserum (entspricht $\frac{2}{3}$ l Ultrafiltrat) können 8 bis 10 mg lyophilisiertes Produkt gewonnen werden. Das so erhaltene rohe Satietin ist als Standardrohprodukt anzusehen, es ist jedoch auch in dieser Form für praktische Zwecke, das heißt zum Regeln des Appetites, durchaus genügend rein. Seine Satietinaktivität beträgt 25 bis 50 S.E./mg.

Die Satietinaktivität des Produktes wird durch eine eigens däfur ausgearbeitete biologische Verfahrensweise gemessen; unter 1 Satietineinheit (S.E.) wird diejenige Wirkstoffmenge, welche bei Verabreichung an 96 Stunden lang ausgehungerte, 200 bis 240 g schwere weibliche CFY-Ratten

intrazerebroventrikulär deren Verzehr von Standardfutterscheiben am ersten Tag der Fütterung von den durchschnittlichen 24,04 ± 0,76 g auf 10 g verringert, verstanden.

Analog wie oben beschrieben kann rohes Satietin nicht nur aus menschlichem Blut, sondern auch aus tierischem Blutserum, wie Blutseren von Rindern, Pferden, Hasen oder Ratten, gewonnen werden. Obwohl die Ausbeute und die Wirkungsstärke des erhaltenen Produktes unterschiedlich sein können (aus dem Serum des Rinderblutes wird zum Beispiel nach demselben Verfahren etwas mehr Produkt [10 bis 13 mg/l] als aus dem Serum von menschlichem Blut erhalten, wobei es die gleiche Wirksamkeit wie die des aus menschlichem Blut gewonnenen Produktes hat), ist der spezifische selektiv den Ernährungsmechanismus ansprechende Charakter der Wirkung bei den Produkten unterschiedlichen Ursprunges der gleiche.

Das mittels Gelelektrophorese mit Natrium-n-dodecylsulfat (SDS) an Polyacrylamid und mittels Gradientenelektrophorese an Polyacrylamidgel bestimmte Molekulargewicht des auf die beschriebene Weise erhaltenen, eine Aktivität von 25 bis 50 S.E./mg aufweisenden rohen Satietines beträgt 50 000 bis 70 000. Es ist salzfrei, enthält praktisch kein Albumin mehr, weist einen geringen Eiweißgehalt (5 bis 25 Gew.-%) und einen hohen Kohlenhydratgehalt (60 bis 90 Gew.-%) auf und ist im lyophilisierten Zustand ein blaßgelbes Pulver. Das Produkt enthält im Kohlenhydratteil die 4 Kauptzuckerbestandteile Fucose, Mannose, Galaktose und Glucose.

Im hydrolysierten Produkt ist in jedem Falle eine beträchtliche Menge Glucosamin nachweisbar. Die mit Natrium-n-dodecylsulfat an Polyacrylamid vorgenommene Gelektrophorese und die analytische isoelektrische Fokussierung zeigen 4 bis 6 durch Eiweißfärben sichtbar werdende Streifen (Bestandteile ode Untereinheiten). Das Produkt wurde ferner in Pufferlösungen mit einem pH-Wert von 6,2 beziehungsweise 1,6 einer Mittelspannungselektrophorese unterzogen und erwies sich dabei noch als Gemisch. Der biologisch aktive Hauptbestandteil bleibt an der Aufgabestelle oder bewegt sich sehr wenig in Richtung der Kathode. Nach Beendigung der Elektrophorese kann der Wirkstoff mit Ninhydrin oder Überjodsäure gleichermaßen nachgewiesen werden, was ebenfalls für die Glucoproteidnatur der Substanz spricht.

Das auf die beschriebene Weise erhaltene rohe Satietin kann elektrophoretisch, im Laboratoriumsmaßstab vorteilhaft durch Papierelektrophorese, weiter gereinigt werden. Bei der vorteilhaft in einer Pufferlösung mit einem pH-Wert von 6,2 vorgenommenen elektrophoretischen Trennung wird das an der Startstelle verbleibende ($R_f$ = 0,1) Hauptprodukt in etwa 75%-iger Ausbeute erhalten, während 2 Begleitbestandteile von eiweißartigem beziehungsweise glucoproteidhaltigem Charakter in Richtung auf die Anode wandern und auf diese Weise völlig vom hochaktiven (60 bis 100 S.E./mg) auf Schiff-Färbung mit Ninhydrin oder Überjodsäure positiv reagierenden Hauptprodukt abgetrennt werden können.

Der auf diese Weise gewonnene Wirkstoff ist schon sehr rein. Bei den durchgeführten Untersuchungen (Gelchromatographie und Gradientenelektrophorese an Polyacrylamidgel) zeigte sich die Substanz als einheitlich. Die Aktivität des im wesentlichen Glucoproteidcharakter zeigenden Stoffes beträgt etwa 100 S.E./mg. Wie jedoch eine leichte Schwankung der Zusammensetzung- und Aktivitätswerte zeigt, ist die Substanz noch nicht als chemisch homogen anzusehen. Die reine Substanz kann erhalten werden, wenn der auf die beschriebene Weise abgetrennte und elektrophoretisch gereinigte Wirkstoff in einer weiteren Trennungsstufe der unlängst entwickelten Affinitätschromatographie (siehe zum Beispiel D. M. Swallow, L. Evans, D. A. Hopkinson: Nature *g269* [1977], 261 bis 262, in welcher Schrifttumsstelle auch sämtliche in diesem Text im Zusammenhang mit der Affinitätschromatographie genannten Substanzen erscheinen) unterzogen wird. Diese kann zweckmäßig wie folgt durchgeführt werden.

Für diese Affinitätschromatographie muß als Adsorbens ein Stoff, welcher eine in der abzutrennenden Substanz vorliegende, in den Begleitstoffen jedoch nicht enthaltene Gruppe selektiv zu binden vermag, verwendet werden. Für das sich als Glucoproteid erwiesene Satietin kann ein Adsorbens, welches die Glucopyranosegruppen der Glucoproteide spezifisch bindet, verwendet werden. Von den bisher bekannten gruppenspezifischen Adsorbentien hat sich das als "Con-A-Sepharose" bezeichnete Adsorbensgel als am vorteilhaftesten erwiesen. Dieses Gel, in welchem an mit Cyanbromid aktivierte Sepharose 4B Concavalin A gekuppelt ist, bindet unter bestimmten Bedingungen die eine α-D-Mannopyranosyl- oder α-D-Glucopyranosylgruppe aufweisenden Moleküle, während die diese Gruppen nicht enthaltenden Begleitstoffe, wie sonstige Eiweiße und Peptide, an das Gel nicht gebunden werden. Diese nicht gebundenen Stoffe erscheinen im ersten Eluat etwa in der $\frac{1}{3}$ des Säulenvolumens entsprechenden Menge. Die an das Gel gebundenen glucoproteidartigen Substanzen können je nach der Art der Bindung, das heißt ihren chemischen Eigenschaften, noch weiter aufgetrennt werden, vor allem, wenn die an das Gel gebundenen Stoffe mit einem Gradienten eluiert werden.

Zur Durchführung der Affinitätschromatographie wird die vorher schon durch Fraktionieren in der angegebenen Weise weitgehend gereinigte Substanz in einer Startpufferlösung von neutraler Reaktion gelöst. Als Startpufferlösung wird vorteilhaft eine wäßrige 0,02 m 2-(Amino)-2-(hydroxymethyl)-propan-1,3-diolhydrochloridlösung [Tris-hydrochloridlösung], welche Natriumchlorid in einer Konzentration von 0,5 bis 1,0 Mol/l und vorteilhaft eine geringe Menge (etwa 1 Millimol) $Ca^{++}$- und $Mn^{++}$-Ionen enthält, verwendet. Die verhältnismäßig hohe Salzkonzentration in der Startpufferlösung ist erforderlich, um eine nicht-spezifische Eiweißbindung zwischen dem Adsorbens und den Begleitstoffen mit Eiweißcharakter zu verhindern. Die in der Ausgangspufferlösung gelöste Substanz wird auf die mit dem Adsorbens, vorzugsweise Con-A-Sepharose, gefüllte Säule aufgebracht. Die Säule wird vorher mit der

Startpufferlösung ins Gleichgewicht gebracht, zum Beispiel in der Weise, daß mindestens das 10-fache Säulenvolumen Pufferlösung durchlaufen gelassen wurde.

Nach dem Aufbringen der Substanz in der Startpufferlösung auf die Säule wird mit einem Konzentrationsgradienten, und zwar einer in steigender Konzentration α-Methylmannosid enthaltenden Pufferlösung, eluiert. Vorteilhaft wird als Eluierlösung eine solche von gleicher Zusammensetzung wie die der Startpufferlösung, aber wie bereits erwähnt mit einem Gehalt an α-Methylmannosid verwendet. Vorzugsweise wird eine α-Methylmannosidlösung mit einer Konzentration von 0,5 Mol/l kontinuierlich der Pufferlösung zugetropft und das Gemisch dauernd gerührt. Auf diese Weise wird ein linearer Gradient erhalten. Während des Eluierens ändert sich nur der Gehalt an α-Methylmannosid, dagegen werden die Ionenkonzentration und der pH-Wert der Lösung während des Eluierens nicht geändert. Wenn der Vorgang bei der Wellenlänge von 254 nm mit einem Registrierphotometer verfolgt wird, wird das in der Zeichnung gezeigte Elutionsdiagramm, gemäß welchem das die Spitze 1 darstellende Stoffgemisch durch die Säule hindurchläuft und in der Anfangsfraktion erscheint, während das an die Säule gebundene Glucoproteid mit einem wesentlich größeren Retentionswert in Form der nahezu symmetrischen Spitze 2 in dem Maße eluiert wird, wie die α-Methylmannosidkonzentration des Gradienten ansteigt. Die entsprechenden Fraktionen werden vereinigt und eingeengt; da die auf diese Weise erhaltene Substanz noch die aus der Pufferlösung stammenden Salze, α-Methylmannosid und gegebenenfalls auch vom hochmolekularen Wirkstoff eventuell abgespaltene niedermolekulare Fragmente enthält, ist zurt Erzielung ded reinen Wirkstoffes eine abschließende Gelchromatographie zu dessen Abtrennung erforderlich. Dazu wird ein Gel mit einem Ausschlußvolumen unter M = 3 000 Dalton, vorteilhaft das Gel "Bio-Gel P-2", verwendet. Es wird mit ionenfreiem Wasser eluiert. Die im Produkt noch enthaltenen Salze und sonstigen niedermolekularen Verunreinigungen treten infolge ihrer geringen Molekülgröße in das innere des Geles ein und werden dort zurückgehalten. Das reine salzfreie Satietin wird von der Oberfläche des Geles gewaschen und erscheint in dem $\frac{1}{3}$ des Säulenvolumens entsprechenden ersten Eluat. Die mit reinem ionenfreiem Wasser erhaltenen wirkstoffhaltigen Fraktionen werden vereinigt und lyophilisiert; auf diese Weise wird der nun völlig reine und einheitliche Wirkstoff Satietin in Form eines weißen Pulvers erhalten.

Die Gewinnung und Reinigung des Wirkstoffes Satietin aus menschlichem oder tierischem Blutserum nach der oben beschriebenen zweckmäßigen Ausführungsform des erfindungsgemäßen Verfahrens ist mit allen Stufen und Zwischenprodukten und den Ausbeuteangaben im folgenden Schema dargestellt.

Menschliches Serum (3 000 $cm^3$)

↓ Ultrafiltration an Amicon® UM-10-Membran

abgetrenntes Eiweiß ← Ultrafiltrat (2 000 $cm^3$)

↓ Konzentrieren, Entfernen des Eiweißes mit einer wäßrigen 55 gew./vol.-%-iger Trichloressigsäurelösung, Ultrazentrifugieren

abgetrennter Niederschlag ← überstehende Flüssigkeit (72 $cm^3$)

↓ Gelchromatographie an Sephadex® G-15 mit einer mit destilliertem Wasser bereiteten 0,1 m Ammoniumacetatpufferlösung

verworfene inaktive Fraktionen ← bei $V_o$ (500 bis 600 $cm^3$) erscheinende aktive Fraktionen

↓ Konzentrieren und Gelchromatographie an Bio-Gel P-2 mit destilliertem Wasser

verworfene inaktive Fraktionen ← bei $V_o$ (130 bis 180 $cm^3$) erscheinende aktive Fraktionen

↓ Lyophilisieren

rohes Satietin, 30 mg

↓ präparative Elektrophorese pH-Wert = 6,2

verworfene inaktive Fraktionen ← aktive Fraktionen, 20 bis 23 mg weißes Pulver

↓ Affinitätschromatographie an Con-A-Sepharose mit einem α-Methylmannosid-Gradienten

homogener aktiver Wirkstoff, 5 bis 6 mg

Wie es auch aus dem obigen Schema hervorgeht, können aus 1 l Blutserum etwa 1,5 bis 2 mg lyophilisiertes Satietin, dessen biologische Aktivität etwa 100 S.E./mg beträgt, hergestellt werden.

Das erfindungsgemäß abgetrennte reine Satietin hat ein Molekulargewicht von 60 000 bis 70 000, wie

es durch Gelektrophorese mit Natrium-n-dodecylsulfat (SDS) festgestellt wurde. An Polyacrylamidgel mit Amfolin mit einem pH-Wert von 5 bis 7 beziehungsweise 7 bis 9 wurde durch isoelektrisches Fokussieren der isoelektrische Punkt zu pl = 7,0 bis 7,1 bestimmt. Die Analyse des sauren Hydrolysates edes Produktes ergab folgende Werte:

| | |
|---|---|
| Eiweiß | 15 Gew.-% |
| Kohlenhydrate | 75 Gew.-% |
| Glucosamin | 4 Gew.-% |
| Wasser | 5 Gew.-% |

Analyse des Aminosäuregehaltes:
L-Asparaginsäure = 1,21 Gew.-%,
L-Threonin = 0,66 Gew.-%, L-Serin = 0,71 Gew.-%,
L-Glutaminsäure = 1,87 Gew.-%,
L-Prolin = 0,45 Gew.-%,
Glykokoll = 0,42 Gew.-%,
L-Alanin = 1,58 Gew.-%,
L-Cystein = 0,13 Gew.-%,
L-Valin = 0,55 Gew.-%,
L-Methionin = 0,12 Gew.-%,
L-Isoleucin = 0,26 Gew.-%, L-Leucin = 0,90 Gew.-%,
L-Tyrosin = 0,31 Gew.-%,
L-Phenylalanin = 0,44 Gew.-%,
Ammoniak = 0,24 Gew.-%, L-Lysin = 3,79 Gew.-%,
L-Histidin = 0,15 Gew.-%, L-Arginin = 0,49 Gew.-%.

Zuckerbestandteile:
Fucose = 19 Gew.-%, Mannose = 21 Gew.-%
Galaktose = 11 Gew.-%,
Glucose = 24 Gew.-%.

Die Analysenwerte beziehen sich auf das lyophilisierte Produkt. Je nach den Umständen der Lyophilisierung kann der Wassergehalt verschieden sein.

Ausweislich der mit Natrium-n-dodecylsulfat vorgenommenen Polyacrylamid-Gelelektrophorese, der Gradienten-Elektrophorese an Polyacrylamidgel und der isoelektrischen Fokussierung handelt es sich um eine chemisch homogene Substanz.

Gegenstand der Erfindung ist auch der Wirkstoff, erhältlich nach dem erfindungsgemäßen Verfahren. Er kann vorteilhaft als Appetitzügler verwendet werden. Er bringt gegenüber dem bekannten unreinen Satietinprodukt den überraschenden großen Vorteil mit sich, daß er 3- bis 4-mal so wirksam wie das letztere ist.

Die Erfindung wird an Hand des folgenden Beispiels näher erläutert.

## Beispiel a

Es wurden 3 000 $cm^3$ menschliches Blutserum unter ständigem Rühren unter einem Druck von 3 bis 4 atm durch eine Amicon® UM-10-Membran filtriert. Das erhaltene Ultrafiltrat (etwa 2 000 $cm^3$) wurde unter Vakuum auf ein Volumen von 60 $cm^3$ eingeengt. Das einen Niederschlag enthaltende Konzentrat wurde mit einer Beschleunigung von 9 000 g 30 Minuten lang zentrifugiert. Die überstehende Flüssigkeit wurde abgetrennt und unter Rühren und Kühlen wurden 12 $cm^3$ einer 55 gew./vol.-%-igen wäßrigen Trichloressigsäurelösung zugeotropft. Das Gemisch wurde bei 5 bis 10°C 1 Stunde lang stehengelassen. Zur Entfernung des gefällten Eiweißes wurde bei etwa 5°C mit einer Es folgen Seiten 16 der Europäischen Offenlegungsschrift 75 925 sowie Auspruchsfassung eingegangen aus 6.2.89 mit den Änderungen gemäß dem Formulierungsvorschlag nach dem Prüfungsbescheid vom 2 Juni, 1989 Beschleunigung von 30 000 ultrazentrifugiert. Die erhaltene Flüssigkeit war optisch völlig klar. Diese Flüssigkeit wurde an einer Säule (5,90 cm) aus Sephadex® G-15 chromatographiert und mit einer mit destilliertem Wasser bereiteten 0,1 m Ammoniumacetatpufferlösung (pH-Wert = 6,6) eluiert. Die Fraktionen zwischen 500 und 600 $cm^3$ wurden vereinigt und lyophilisiert. Der lyophilisierte Rückstand wurde in 10 $cm^3$ Wasser gelöst und auf eine Gelsäule aus Bio-Gel P-2 (2,5 × 90 cm) aufgebracht. Die Säule wurde mit destilliertem Wasser eluiert und die Fraktionen zwischen 130 und 180 $cm^3$ wurden vereinigt und lyophilisiert. Auf diese Weise wurden 25 bis 30 mg rohes salzfreies Satietin in Form eines weißlichgelben Pulvers erhalten.

## Beispiel b

30 mg des wie unter dem vorstehenden Abschnit a) beschrieben erhaltenen Rohproduktes wurden in destilliertem Wasser zu einer Lösung mit einer Konzentration von 5 mg/$cm^3$ gelöst. Diese Lösung wurde in einem 2 cm breiten und 30 cm langen Streifen auf ein Elektrophoresepapier vom Typ Whatman® 3M

aufgetropft. Das Papier wurde getrocknet und dann mit einer Pufferlösung mit einem pH-Wert von 6,2 (10 Vol.-% Pyridin und 0,5 Vol.-% Essigsäure) gleichmäßig angefeuchtet und in einem horizontalen Gerät bei einem Spannungsgefälle von 20 V/cm 4 Stunden lang der Elektrophorese unterzogen. Dann wurde das Papier getrocknet und auf einem 1 cm breiten Streifen mit Ninhydrin beziehungsweise überjodsaurem Schiff-Reagens angefärbt, wodurch die getrennten Bestandteile sichtbar wurden. Dann wurde das Papier zerschnitten und aus den einzelnen Teilen wurde der Wirkstoff mit destilliertem Wasser herausgelöst. Nach dem Lyophilisieren wurden 20 bis 23 mg Wirkstoff mit einer Aktivität von 50 bis 100 S.E./mg erhalten.

Beispiel c

Es wurden 20 mg des wie unter dem vorstehenden Abschnitt b) beschrieben elektrophoretisch gereinigten Produktes in 4 $cm^3$ einer wäßrigen Startpufferlösung mit einem Gehalt an 0,02 Mol/l 2-(Amino)-2-(hydroxymethyl)propan-1,3-diolhydrochlorid [Trishydrochlorid], 0,5 Mol/1 Natriumchlorid, 0,001 Mol/l Calciumchlorid und 0,001 Mol/l Manganchlorid und mit einem pH-Wert von 7,0 auf eine mit Con-A-Sepharose gefüllte Säule (Durchmesser = 1,7 cm und Länge = 37 cm) aufgebracht. Die Säule ist vorher mit einer dem Säulenvolumen entsprechenden Menge der gleichen Pufferlösung durchgespült worden. Das Eluieren wurde mit 100 $cm^3$ der Startpufferlösung begonnen. Dieser wurden unter ständigem Rühren 100 $cm^3$ einer Pufferlösung der gleichen Zusammensetzung, jedoch darüberhinaus noch mit einem Gehalt an 0,5 Mol/l α-Methylmannosid zugetropft. Im verwendeten Gradienten änderte sich demnach die α-Methylmannosidkonzentration linear von 0 bis 0,5 Mol/l. Der Vorgang wurde auf einem UV-Monitor (Wellenlänge 254 nm) kontinuierlich verfolgt. Es wurden Fraktionen zu je 2,5 $cm^3$ aufgefangen. Die das gewünschte Produkt enthaltenden Fraktionen (zwischen 75 und 100 $cm^3$) wurden verinigt und durch Lyophilisieren auf ein Volumen von 10 $cm^3$ eingeengt.

Die konzentrierte Flüssigkeit wurde zur Entfernung der Salze und sonstigen niedermolekularen Verunreinigungen auf eine mit Bio-Gel P-2 gefüllte Säule (Durchmesser = 2,5 cm, und Länge = 90 cm) aufgebracht und mit ionenfreiem Wasser eluiert. Die Fraktionen zwischen 140 und 180 $cm^3$ wurden vereinigt und lyophilisiert.

So wurden 5 bis 6 mg Satietin als schneeweißes, salzfreies, homogenes Produkt erhalten. Die Aktivität dieses Produktes begrug 100 S.E./mg.

**Patentansprüche für die Vertragsstaaten: BE CH DE FR GB IT LI NL SE**

1. Verfahren zur Herstellung eines spezifisch auf das Ernährungszentrum wirkenden appetitregelnden Wirkstoffes aus menschlichem und/oder tierischem Blutserum durch dessen Membranfiltrieren durch ein bis zu einem Molekulargewicht von 50 000 durchlässiges Membranfilter und Eindampfen des Filtrates sowie Gelchromatographieren einer Lösung der erhaltenen Materiales an einem Gel mit einem Ausschlußvolumen M unter 50 000 Dalton und fraktioniertes Eluieren mit einer wäßrigen Lösung, erneutes Gelchromatographieren einer Lösung der konzentrierten biologisch aktiven Fraktionen an einem Gel mit einem Ausschlußvolumen M unter 50 000 Dalton und fraktioniertes Eluieren mit Wasser und Konzentrieren der biologisch aktiven Fraktionen, dadurch gekennzeichnet, daß man das beim Membranfiltrieren erhaltene Filtrat nur partiell eindampft und aus dem erhaltenen Konzentrat den unlöslichen Teil entfernt, dann zur flüssigen Phase bei 0 bis 10°C eine wäßrige Trichloressigsäurelösung bis zum Erreichen einer Trichloressigsäurekonzentration von 5 bis 25 Gew./Vol-% zugibt und das ausgefallene Eiweiß entfernt sowie das Gelchromatographieren der erhaltenen Lösung an einem Gel mit einem Ausschlußvolumen unter M = 4 000 Dalton durchführt und das Eluieren mit einer 0,5 bis 1,0 gew.-%-igen wäßrigen Natriumchloridlösung oder einer wäßrigen Pufferlösung mit einem pH-Wert von 6,0 bis 7,0 vornimmt, das Konzentrieren der biologisch aktiven Fraktionen durch Lyophilisieren bewerkstelligt, das erneute Gelchromatographieren des erhaltenen Konzentrates an einem Gel mit einem Ausschlußvolumen unter M = 3 000 Dalton durchführt und das Konzentrieren der mit Wasser eluierten biologisch aktiven Fraktionen durch Lyophilisieren vornimmt sowie das erhaltene Produkt einer weiteren Reinigung in der Weise unterzieht, daß man eine bei einem pH-Wert von 6,0 bis 6,5 vorgenommene Elektrophorese durchführt und das an der Startstelle gebliebene Hauptprodukt abtrennt und unter Verwendung eines in Bezug auf Glucopyranose- und/oder Mannopyranosegruppen eine spezifische Affinität zeigenden Adsorbens einer Affinitätschromatographie unterzieht.

2. Verfahren nach Anspruch 1, dadurch gekennzeichnet, daß man als Adsorbens für die Affinitätschromatographie Con-A-Sepharose verwendet.

3. Verfahren nach Anspruch 1 oder 2, dadurch gekennzeichnet, daß man des Entfernen des unlöslichen Teiles des nach dem partiellen Eindampfen des Filtrates des Membranfiltrierens erhaltenen Konzentrates durch Zentrifugieren durchführt.

4. Verfahren nach Anspruch 1 bis 3, dadurch gekennzeichnet, daß man die Zugabe der Trichloressigsäurelösung bis zum Erreichen einer Konzentration von 10 bis 12 Gew./Vol.-% vornimmt.

5. Verfahren nach Anspruch 1 bis 4, dadurch gekennzeichnet, daß man das Entfernen des auf die Zugabe der Trichloressigsäurelösung hin ausgefallenen Eiweißes durch Ultrazentrifugieren durchführt.

6. Verfahren nach Anspruch 1 bis 5, dadurch gekennzeichnet, daß man zum Eluieren als Natriumchloridlösung eine solche mit einer Konzentration von 0,9 Gew.-% verwendet.

7. Wirkstoff, erhältlich nach dem Verfahren gemäß den Ansprüchen 1 bis 6.

**Patentansprüche für den Vertragsstaat: AT**

1. Verfahren zur Herstellung eines spezifisch auf das Ernährungszentrum wirkenden appetitregelnden Wirkstoffes aus menschlichem und/oder tierischem Blutserum durch dessen Membranfiltrieren durch ein bis zu einem Molekulargewicht von 50 000 durchlässiges Membranfilter und Eindampfen des Filtrates sowie Gelchromatographieren einer Lösung der erhaltenen Materiales an einem Gel mit einem Ausschlußvolumen M unter 50 000 Dalton und fraktioniertes Eluieren mit einer wäßrigen Lösung, erneutes Gelchromatographieren einer Lösung der konzentrierten biologisch aktiven Fraktionen an einem Gel mit einem Ausschlußvolumen M unter 50 000 Dalton und fraktioniertes Eluieren mit Wasser und Konzentrieren der biologisch aktiven Fraktionen, dadurch gekennzeichnet, daß man das beim Membranfiltrieren erhaltene Filtrat nur partiell eindampft und aus dem erhaltenen Konzentrat den unlöslichen Teil entfernt, dann zur flüssigen Phase bei 0 bis 10°C eine wäßrige Trichloressigsäurelösung bis zum Erreichen einer Trichloressigsäurekonzentration von 5 bis 25 Gew./Vol-% zugibt und das ausgefallene Eiweiß entfernt sowie das Gelchromatographieren der erhaltenen Lösung an einem Gel mit einem Ausschlußvolumen unter M = 4 000 Dalton durchführt und das Eluieren mit einer 0,5 bis 1,0 gew.-%-igen wäßrigen Natriumchloridlösung oder einer wäßrigen Pufferlösung mit einem pH-Wert von 6,0 bis 7,0 vornimmt, das Konzentrieren der biologisch aktiven Fraktionen durch Lyophilisieren bewerkstelligt, das erneute Gelchromatographieren des erhaltenen Konzentrates an einem Gel mit einem Ausschlußvolumen unter M = 3 000 Dalton durchführt und das Konzentrieren der mit Wasser eluierten biologisch aktiven Fraktionen durch Lyophilisieren vornimmt sowie das erhaltene Produkt einer weiteren Reinigung in der Weise unterzieht, daß man eine bei einem pH-Wert von 6,0 bis 6,5 vorgenommene Elektrophorese durchführt und das an der Startstelle gebliebene Hauptprodukt abtrennt und unter Verwendung eines in Bezug auf Glucopyranose- und/oder Mannopyranosegruppen eine spezifische Affinität zeigenden Adsorbens einer Affinitätschromatographie unterzieht.

2. Verfahren nach Anspruch 1, dadurch gekennzeichnet, daß man als Adsorbens für die Affinitätschromatographie Con-A-Sepharose verwendet.

3. Verfahren nach Anspruch 1 oder 2, dadurch gekennzeichnet, daß man des Entfernen des unlöslichen Teiles des nach dem partiellen Eindampfen des Filtrates des Membranfiltrierens erhaltenen Konzentrates durch Zentrifugieren durchführt.

4. Verfahren nach Anspruch 1 bis 3, dadurch gekennzeichnet, daß man die Zugabe der Trichloressigsäurelösung bis zum Erreichen einer Konzentration von 10 bis 12 Gew./Vol.-% vornimmt.

5. Verfahren nach Anspruch 1 bis 4, dadurch gekennzeichnet, daß man das Entfernen des auf die Zugabe der Trichloressigsäurelösung hin ausgefallenen Eiweißes durch Ultrazentrifugieren durchführt.

6. Verfahren nach Anspruch 1 bis 5, dadurch gekennzeichnet, daß man zum Eluieren als Natriumchloridlösung eine solche mit einer Konzentration von 0,9 Gew.-% verwendet.

**Revendications pour les Etats contractants: BE CH DE FR GB IT LI NL SE**

1. Procédé pour préparer une substance active agissant de façon spécifique sur le centre de la nutrition et réglant l'appétit, à partir d'un sérum sanguin humain et/ou animal au moyen d'une filtration à travers une membrane à l'aide d'un filtre à membrane perméable jusqu'à un poids moléculaire de 50 000, et d'une évaporation du filtrat ainsi qu'une chromatographie d'une solution de la substance obtenue, sur un gel possédant un volume d'exclusion M inférieur à 50 000 daltons, et d'une élution fractionnée avec une solution aqueuse, d'une nouvelle chromatographie sur gel d'une solution concentrée, active du point de vue biologique, sur un gel avec un volume d'exclusion M inférieur à 50 000 daltons et d'une élution fractionnée avec de l'eau, et d'une concentration des fractions actives du point de vue biologique, caractérisé en ce qu'on évapore seulement partiellement le filtrat obtenu lors de la filtration à l'aide de la membrane et qu'on élimine, du concentré obtenu, la partie insoluble, qu'on ajoute ensuite à la phase liquide, entre 0 et 10°C, une solution d'acide trichloracétique aqueux jusqu'à l'obtention d'une concentration de 5 à 25% en poids/volume de l'acide trichloracétique, et qu'on élimine la protéine qui a précipité, et qu'on exécute la chromatographie de la solution obtenue sur un gel possédant un volume d'exclusion inférieur à M = 4 000 daltons et qu'on réalise l'élution avec une solution de chlorure de sodium aqueuse intervenant pour 0,5 à 1,0% en poids ou avec une solution tampon aqueuse possédant un pH compris entre 6,0 à 7,0, que l'on concentre les fractions, actives du point de vue biologique, par liophylisation, qu'on exécute une nouvelle chromatographie du concentré obtenu sur un gel possédant un volume d'exclusion à M = 3 000 daltons et que l'on concentre les fractions éluées avec l'eau et actives du point de vue biologique, par lyophilisation et qu'on soumet le produit obtenu à une épuration ultérieure de manière à exécuter une électrophorèse réalisée avec un pH possédant une valeur comprise entre 6,0 et 6,5, et qu'on sépare le produit principal resté au point de départ et qu'on le soumet à une chromatographie par affinité, moyennant l'utilisation d'un agent adsorbant présentant une affinité spécifique vis-à-vis de groupes glucopyranose et/ou mannopyranose.

2. Procédé selon la revendication 1, caractérisé en ce qu'on utilise de la Con-A-Sépharose.

3. Procédé selon la revendication 1 ou 2, caractérisé en ce qu'on réalise par centrifugation l'élimination de la partie insoluble du concentrat obtenu après l'évaporation partielle du filtrat fourni par la filtration à travers la membrane.

4. Procédé selon l'une des revendications 1 à 3, caractérisé en ce qu'on réalise l'adjonction de la solution d'acide trichloracétique jusqu'à l'obtention d'une concentration comprise entre 10 et 12% en poids/volume.

5. Procédé selon la revendications 1 à 4, caractérisé en ce qu'on exécute par centrifugation l'élimination de la protéine qui précipite lors de l'adjonction de l'acide trichloracétique.

6. Procédé selon l'une des revendications 1 à 5, caractérisé en ce que pour l'élution, on utilise comme solution de chlorure de sodium, une telle solution possédant une concentration de 0,9% en poids.

7. Substance active pouvant être obtenue à l'aide du procédé selon les revendications 1 à 6.

**Revendications pour l'Etat contractant: AT**

1. Procédé pour préparer une substance active agissant de façon spécifique sur le centre de la nutrition et réglant l'appétit, à partir d'un sérum sanguin humain et/ou animal au moyen d'une filtration à travers une membrane à l'aide d'un filtre à membrane perméable jusqu'à un poids moléculaire de 50 000, et d'une évaporation du filtrat ainsi qu'une chromatographie d'une solution de la substance obtenue, sur un gel possédant un volume d'exclusion M inférieur à 50 000 daltons, et d'une élution fractionnée avec une solution aqueuse, d'une nouvelle chromatographie sur gel d'une solution concentrée, active du point de vue biologique, sur un gel avec un volume d'exclusion M inférieur à 50 000 daltons et d'une élution fractionnée avec de l'eau, et d'une concentration des fractions actives du point de vue biologique, caractérisé en ce qu'on évapore seulement partiellement le filtrat obtenu lors de la filtration à l'aide de la membrane et qu'on élimine, du concentré obtenu, la partie insoluble, qu'on ajoute ensuite à la phase liquide, entre 0 et 10°C, une solution d'acide trichloracétique aqueux jusqu'à l'obtention d'une concentration de 5 à 25% en poids/volume de l'acide trichloracétique, et qu'on élimine la protéine qui a précipité, et qu'on exécute la chromatographie de la solution obtenue sur un gel possédant un volume d'exclusion inférieur à M = 4 000 daltons et qu'on réalise l'élution avec une solution de chlorure de sodium aqueuse intervenant pour 0,5 à 1,0% en poids ou avec une solution tampon aqueuse possédant un pH compris entre 6,0 à 7,0, que l'on concentre les fractions, actives du point de vue biologique, par liophylisation, qu'on exécute une nouvelle chromatographie du concentré obtenu sur un gel possédant un volume d'exclusion à M = 3 000 daltons et que l'on concentre les fractions éluées avec l'eau et actives du point de vue biologique, par lyophilisation et qu'on soumet le produit obtenu à une épuration ultérieure de manière à exécuter une électrophorèse réalisée avec un pH possédant une valeur comprise entre 6,0 et 6,5, et qu'on sépare le produit principal resté au point de départ et qu'on le soumet à une chromatographie par affinité, moyennant l'utilisation d'un agent adsorbant présentant une affinité spécifique vis-à-vis de groupes glucopyranose et/ou mannopyranose.

2. Procédé selon la revendication 1, caractérisé en ce qu'on utilise de la Con-A-Sépharose.

3. Procédé selon la revendication 1 ou 2, caractérisé en ce qu'on réalise par centrifugation l'élimination de la partie insoluble du concentrat obtenu après l'évaporation partielle du filtrat fourni par la filtration à travers la membrane.

4. Procédé selon l'une des revendications 1 à 3, caractérisé en ce qu'on réalise l'adjonction de la solution d'acide trichloracétique jusqu'à l'obtention d'une concentration comprise entre 10 et 12% en poids/volume.

5. Procédé selon la revendications 1 à 4, caractérisé en ce qu'on exécute par centrifugation l'élimination de la protéine qui précipite lors de l'adjonction de l'acide trichloracétique.

6. Procédé selon l'une des revendications 1 à 5, caractérisé en ce que pour l'élution, on utilise comme solution de chlorure de sodium, une telle solution possédant une concentration de 0,9% en poids.

**Claims for the Contracting States: BE CH DE FR GB IT LI NL SE**

1. A process for the preparation of an appetite-regulating active ingredient acting specifically on the nutrition centre from human and/or animal blood serum by membrane filtration thereof through a membrane filter that is permeable up to a molecular weight of 50,000 and by concentration of the filtrate by evaporation, and by gel chromatography of a solution of the resulting material on a gel having an exclusion volume M below 50,000 Daltons and fractional elution with an aqueous solution, by further gel chromatography of a solution of the concentrated biologically active fractions on a gel having an exclusion volume M below 50,000 Daltons and fractional elution with water and concentration of the biologically active fractions, characterised in that the filtrate obtained after membrane filtration is only partially concentrated by evaporation, and the insoluble portion is removed from the resulting concentrate and then an aqueous trichloroacetic acid solution is added to the fluid phase at from 0° to 10° until a trichloroacetic acid concentration of from 5 to 25% weight/volume is obtained, and the precipitated protein is removed, and the gel chromatography of the resulting solution is carried out on a gel having an exclusion volume below M = 4,000 Daltons and the elution is carried out with a 0.5 to 1.0% by weight aqueous sodium chloride solution or an aqueous buffer solution having a pH value of from 6.0 to 7.0, the concentration of the biologically active fractions is effected by lyophilisation, the further gel chromatography of the resulting concentrate is carried out on a gel having an exclusion volume below M = 3,000 Daltons and the concentration of the biologically active fractions eluted with water is carried out by lyophilisation, and the

resulting product is subjected to further purification by carrying out electrophoresis at a pH value of from 6.0 to 6.5 and separating off the main product that has remained at the starting point and subjecting it to affinity chromatography using an adsorbent exibiting a specific affinity with respect to glucopyranose and/or mannopyranose groups.

2. A process according to claim 1, characterised in that the adsorbent used for the affinity chromatography is Con-A-sepharose.

3. A process according to claim 1 or 2, characterised in that the removal of the insoluble portion of the concentrate obtained after the partial concentration by evaporation of the filtrate from the membrane filtration is carried out by centrifugation.

4. A process according to claims 1 to 3, characterised in that the trichloroacetic acid solution is added until a concentration of from 10 to 12% weight/volume is obtained.

5. A process according to claims 1 to 4, characterised in that the removal of the protein precipitated after the addition of the trichloroacetic acid solution is effected by ultracentrifugation.

6. A process according to claims 1 to 5, characterised in that the sodium chloride solution used for elution is a sodium chloride solution having a concentration of 0.9% by weight.

7. An active ingredient obtainable by the process according to claims 1 to 6.

**Claims for the Contracting State: AT**

1. A process for the preparation of an appetite-regulating active ingredient acting specifically on the nutrition centre from human and/or animal blood serum by membrane filtration thereof through a membrane filter that is permeable up to a molecular weight of 50,000 and by concentration of the filtrate by evaporation, and by gel chromatography of a solution of the resulting material on a gel having an exclusion volume M below 50,000 Daltons and fractional elution with an aqueous solution, by further gel chromatography of a solution of the concentrated biologically active fractions on a gel having an exclusion volume M below 50,000 Daltons and fractional elution with water and concentration of the biologically active fractions, characterised in that the filtrate obtained after membrane filtration is only partially concentrated by evaporation, and the insoluble portion is removed from the resulting concentrate and then an aqueous trichloroacetic acid solution is added to the fluid phase at from 0° to 10° until a trichloroacetic acid concentration of from 5 to 25% weight/volume is obtained, and the precipitated protein is removed, and the gel chromatography of the resulting solution is carried out on a gel having an exclusion volume below $M = 4{,}000$ Daltons and the elution is carried out with a 0.5 to 1.0% by weight aqueous sodium chloride solution or an aqueous buffer solution having a pH value of from 6.0 to 7.0, the concentration of the biologically active fractions is effected by lyophilisation, the further gel chromatography of the resulting concentrate is carried out on a gel having an exclusion volume below $M = 3{,}000$ Daltons and the concentration of the biologically active fractions eluted with water is carried out by lyophilisation, and the resulting product is subjected to further purification by carrying out electrophoresis at a pH value of from 6.0 to 6.5 and separating off the main product that has remained at the starting point and subjecting it to affinity chromatography using an adsorbent exibiting a specific affinity with respect to glucopyranose and/or mannopyranose groups.

2. A process according to claim 1, characterised in that the adsorbent used for the affinity chromatography is Con-A-sepharose.

3. A process according to claim 1 or 2, characterised in that the removal of the insoluble portion of the concentrate obtained after the partial concentration by evaporation of the filtrate from the membrane filtration is carried out by centrifugation.

4. A process according to claims 1 to 3, characterised in that the trichloroacetic acid solution is added until a concentration of from 10 to 12% weight/volume is obtained.

5. A process according to claims 1 to 4, characterised in that the removal of the protein precipitated after the addition of the trichloroacetic acid solution is effected by ultracentrifugation.

6. A process according to claims 1 to 5, characterised in that the sodium chloride solution used for elution is a sodium chloride solution having a concentration of 0.9% by weight.

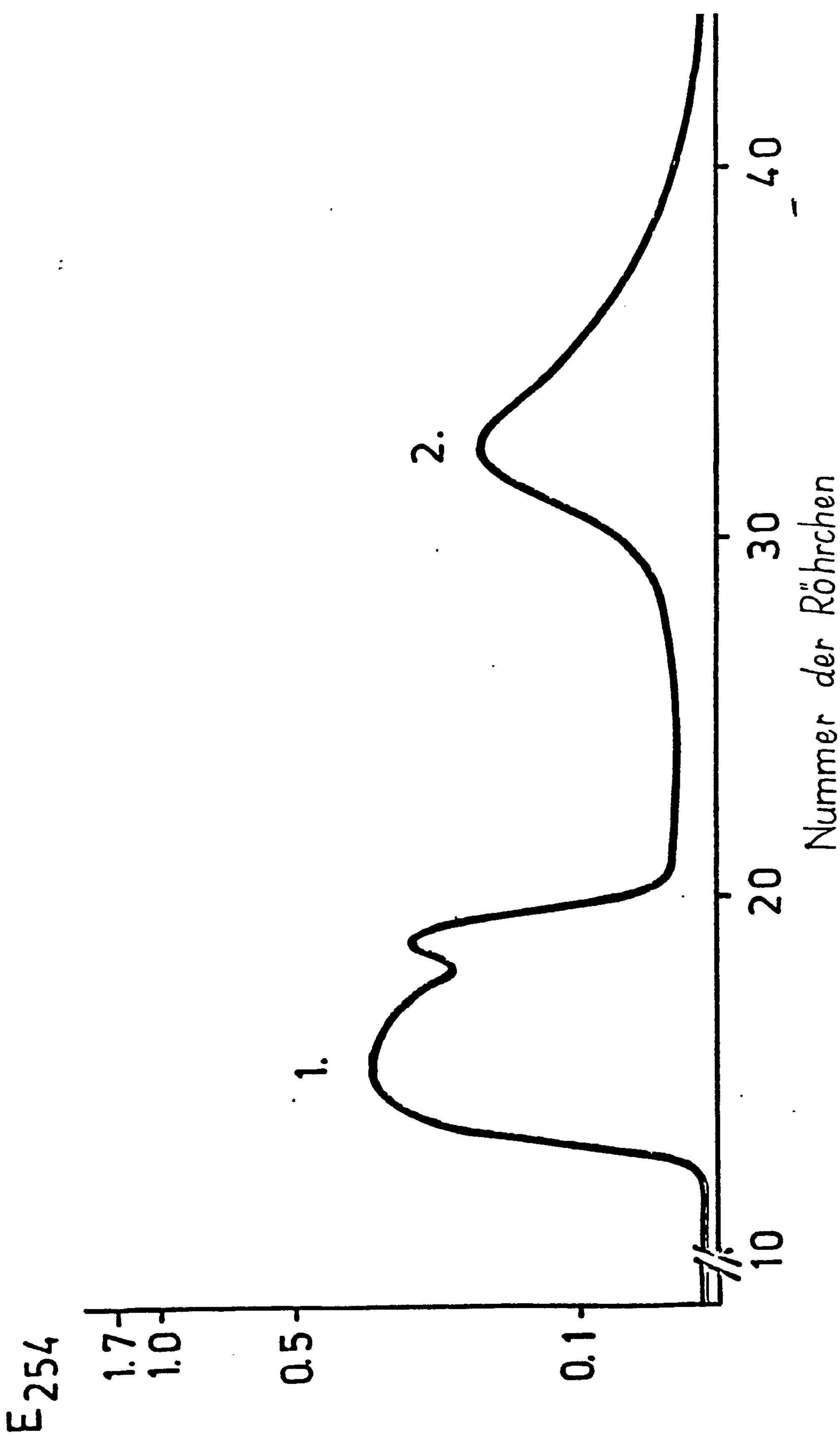


Affinitätschromatographie an einer Con-A-Sepharose-Säule